(19) Europäisches Patentamt
European Patent Office
Office européen des brevets

(11) **EP 4 455 262 A1**

(12) **EUROPEAN PATENT APPLICATION**
published in accordance with Art. 153(4) EPC

(43) Date of publication:
**30.10.2024 Bulletin 2024/44**

(21) Application number: **22910657.0**

(22) Date of filing: **11.11.2022**

(51) International Patent Classification (IPC):
**C12M 3/00** (2006.01)

(52) Cooperative Patent Classification (CPC):
**C12M 1/14; C12M 3/00; C12N 5/06**

(86) International application number:
**PCT/JP2022/042045**

(87) International publication number:
**WO 2023/119938 (29.06.2023 Gazette 2023/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **22.12.2021 JP 2021207731**

(71) Applicant: **Toyo Seikan Group Holdings, Ltd.
Shinagawa-ku
Tokyo 141-8627 (JP)**

(72) Inventors:
• **OTSUKI Azusa
Yokohama-shi, Kanagawa 240-0062 (JP)**
• **KUNINORI Masahiro
Yokohama-shi, Kanagawa 240-0062 (JP)**
• **ICHIKAWA Kentarou
Yokohama-shi, Kanagawa 240-0062 (JP)**

(74) Representative: **Hasegawa, Kan
Patentanwaltskanzlei Hasegawa
Untere Hauptstraße 56
85354 Freising (DE)**

(54) **CELL CULTURE MEMBRANE, AND METHOD FOR PRODUCING BIOLOGICAL TISSUE**

(57) The present invention makes it possible to provide a cell culture membrane capable of easily decomposing after allowing cell layers to be formed on both sides of the cell culture membrane. A cell culture membrane having a cell culture region at both surfaces thereof, the cell culture membrane containing methyl cellulose having a weight average molecular weight of from 20000 to 190000 as a main component, or containing methyl cellulose having a weight average molecular weight of from 190000 to 400000 as a main component, and further containing a dissolution temperature regulator. The dissolution temperature regulator is preferably at least one of sodium styrenesulfonate, sodium iodide, or sodium toluenesulfonate. A poorly soluble material is preferably added to the cell culture membrane.

FIG. 1

**Description**

Technical Field

The present invention relates to a cell culture membrane used for forming a plurality of cell layers, and a method for producing a biological tissue using the cell culture membrane.

Background Art

In recent years, for the production of a biological tissue, double-sided culture has been performed. In the double-sided culture, both surfaces of a cell culture membrane as culture regions are used to perform cell culture, which can produce a biological tissue including a plurality of cell layers. Due to the capability of allowing formation of a plurality of cell layers containing cells of different types from each other, the double-sided culture is useful for, for example, analysis of interaction between different types of cells.

The double-sided culture can be performed using, for example, an organ chip. The organ chip is formed by stacking two substrates and a cell culture membrane, a flow path (micro flow path) is formed in each of the substrates, the flow paths are separated from each other by the cell culture membrane, and cells that are the same or different between the flow paths can be cultured in the flow paths.

Citation List

Patent Literature

Patent Document 1: JP 2017-504320 T

Summary of Invention

Technical Problem

In a resin-made semipermeable membrane widely used as a cell culture membrane, pores of the membrane are gradually blocked by the formation of cell layers, and thus, the cell culture membrane has a decreased permeability of a liquid component which may permeate through the cell culture membrane. As a result, there is an issue in which the semipermeable membrane cannot be appropriately used for the above-mentioned analysis of interaction or the like.

Meanwhile, when a cell culture membrane having a large pore diameter (for example, 10 $\mu$m or more) is used, there is a problem that cells pass through the cell culture membrane when being seeded, and a cell layer cannot be appropriately formed on both sides of the cell culture membrane.

There is also an issue in which the cell layers formed on the cell culture membrane are difficult to separate and take out from the cell culture membrane.

For example, when enzyme treatment is performed using an enzyme such as trypsin, the cells are scattered and cannot be separated from the cell culture membrane as cell layers.

Meanwhile, it is possible to allow cell layers to be formed on a cell culture membrane containing poly(N-isopropylacrylamide) (pNIPAM), and separate the cell layers from the cell culture membrane by dissolving pNIPAM without performing enzyme treatment. However, dissolution of pNIPAM has an issue in which toxicity of the monomer is concerned.

Therefore, the present inventors have made intensive studies and succeeded in developing a cell culture membrane capable of solving the above-mentioned issues. Specifically, the cell culture membrane contains methyl cellulose as a main component. The cell culture membrane does not hinder permeation of a liquid component between cell layers because methyl cellulose is dissolved by temperature drop after the cell layers are formed on the cell culture membrane. With the cell culture membrane, there is no possibility that toxicity occurs when the cell layers are separated from the cell culture membrane.

Incidentally, there is an issue in which dissolution of methyl cellulose usually takes one hour or more. However, when the formed cell layers are used for, for example, analysis of interaction between different types of cells, methyl cellulose is desirably dissolved as quickly as possible, and methyl cellulose is desirably dissolved in 10 minutes or less. This is because the longer the period of time in which the cells are left in a low-temperature environment is, the greater the damage to the cells is.

Therefore, the present inventors have further made studies, and specified a weight average molecular weight of methyl cellulose and a temperature range with which methyl cellulose can be dissolved in 10 minutes or less. Moreover, as for methyl cellulose having a weight average molecular weight equal to or larger than the above-mentioned range, the present inventors have succeeded in specifying a weight average molecular weight of methyl cellulose and a temperature range with which methyl cellulose can be dissolved in 10 minutes or less, with a cell culture membrane containing a dissolution temperature regulator.

Here, Patent Document 1 describes that in a low-shear microfluidic device, a biocompatible material that dissolves over time is used as a cell culture membrane.

However, in such a technique in which the cell culture membrane dissolves over time, the cell layers cannot be appropriately used for, for example, analysis of interaction between different types of cells, and the technique cannot be used for separating the cell layers from the cell culture membrane.

The present invention has been made in view of the above-mentioned circumstances, and an object of the present invention is to provide a cell culture membrane capable of easily decomposing after allowing cell layers to be formed on both sides of the cell culture membrane, and a method for producing a biological tissue using the cell culture membrane.

Solution to Problem

In order to achieve the above-mentioned object, a cell culture membrane of the present invention has a cell culture region at both surfaces thereof, and contains methyl cellulose having a weight average molecular weight of from 20000 to 190000 as a main component.

In addition, the cell culture membrane of the present invention has a cell culture region at both surfaces thereof, contains methyl cellulose having a weight average molecular weight of from 190000 to 400000 as a main component, and further contains a dissolution temperature regulator.

In the cell culture membrane of the present invention, it is preferable that the dissolution temperature regulator be at least one of sodium styrenesulfonate, sodium iodide, or sodium toluenesulfonate.

In the cell culture membrane of the present invention, it is preferable that the poorly soluble material be at least one of polyethylene terephthalate, a glass fiber, a cellulose ester, polyvinylidene fluoride, or polycarbonate.

In the cell culture membrane of the present invention, it is preferable that the poorly soluble material be at least one of polystyrene, a cycloolefin polymer, or a cycloolefin copolymer.

In addition, it is more preferable that the cell culture membrane have a scaffold material for cells on at least one surface thereof, and it is still more preferable that the scaffold material be at least one of collagen, Matrigel, fibronectin, laminin, chitosan, or silk.

It is more preferable that at least one surface of the cell culture membrane be subjected to surface treatment.

It is also preferable that the cell culture membrane of the present invention contain adhesive cells.

In addition, a method for producing a biological tissue of the present invention is a method for producing a biological tissue including a plurality of cell layers, the method including: supplying cells and a culture solution to two culture spaces of a biological tissue forming device including a cell culture membrane and the two culture spaces, the cell culture membrane having a cell culture region at both surfaces thereof, the cell culture membrane being disposed between a plurality of cell layers after the cells are cultured, and the cell culture membrane containing methyl cellulose having a weight average molecular weight of from 20000 to 190000 as a main component, and the two culture spaces being divided by the cell culture membrane; culturing the cells in the two culture spaces to form cell layers on both surfaces of the cell culture membrane; and dissolving methyl cellulose in the cell culture membrane at 15°C or lower.

In addition, a method for producing a biological tissue of the present invention is a method for producing a biological tissue including a plurality of cell layers, the method including: supplying cells and a culture solution to two culture spaces of a biological tissue forming device including a cell culture membrane and the two culture spaces, the cell culture membrane having a cell culture region at both surfaces thereof, the cell culture membrane being disposed between a plurality of cell layers after the cells are cultured, the cell culture membrane containing methyl cellulose having a weight average molecular weight of from 190000 to 400000 as a main component, and the cell culture membrane further containing a dissolution temperature regulator, and the two culture spaces being divided by the cell culture membrane; culturing the cells in the two culture spaces to form cell layers on both surfaces of the cell culture membrane; and dissolving methyl cellulose in the cell culture membrane at 20°C or lower.

In the method for producing a biological tissue of the present invention, it is preferable that methyl cellulose in the cell culture membrane be dissolved in 10 minutes or less.

In addition, it is preferable that the method for producing a biological tissue of the present invention be a method in which a poorly soluble material is added to the cell culture membrane, and the cell layers are supported by the poorly soluble material after dissolution of methyl cellulose.

Advantageous Effects of Invention

According to the present invention, it is possible to provide a cell culture membrane capable of easily decomposing after allowing cell layers to be formed on both sides of the cell culture membrane, and a method for producing a biological tissue using the cell culture membrane.

Brief Description of Drawings

FIG. 1 shows schematic views illustrating a process of forming cell layers using a cell culture membrane according to a first embodiment and a second embodiment of the present invention.

FIG. 2 shows schematic views illustrating a process of forming cell layers using a cell culture membrane according to a third embodiment of the present invention.

FIG. 3 shows schematic views illustrating a process of producing a cell culture membrane (a first method) according to the third embodiment of the present invention.

FIG. 4 shows schematic views illustrating a process of producing a cell culture membrane (a second method) according to the third embodiment of the present invention.

FIG. 5 is a schematic view illustrating constituent members of a microfluidic device in which the cell culture membrane according to each embodiment of the present invention can be used.

FIG. 6 is a schematic view illustrating a structure of the microfluidic device in which the cell culture membrane according to each embodiment of the present invention can be used.

FIG. 7 is a schematic view illustrating a partial cross section of the microfluidic device in which the cell culture membrane according to each embodiment of the present invention can be used.

FIG. 8 is a table showing the results of Experiment 1 performed to confirm the dissolution conditions of the cell culture membranes according to the first embodiment and the second embodiment of the present invention.

FIG. 9 is a table showing the results of Experiment 2 performed to confirm the dissolution conditions of the cell culture membranes according to the first embodiment and the second embodiment of the present invention.

FIG. 10 is a table showing the results of Experiment 3 performed to confirm the dissolution conditions of the cell culture membranes according to the first embodiment and the second embodiment of the present invention.

FIG. 11 shows schematic views illustrating a process of forming cell layers using a known cell culture membrane.

Description of Embodiments

Hereinafter, embodiments of the cell culture membrane and the method for producing a biological tissue of the present invention are described in detail. However, the present invention is not limited to the specific contents of the following embodiments and examples.

First Embodiment

First, a cell culture membrane according to a first embodiment of the present invention is described.

The cell culture membrane of the present embodiment is a temperature-responsive cell culture base material, and can be dissolved by being kept at a certain temperature or lower for a predetermined time.

Specifically, the cell culture membrane of the present embodiment has a cell culture region at both surfaces thereof, and contains methyl cellulose having a weight average molecular weight (Mw) of from 20000 to 190000 as a main component.

The cell culture membrane of the present embodiment is formed into a film shape, and both surfaces of the cell culture membrane are used as a cell culture region (culture portion).

Methyl cellulose is a material excellent in biocompatibility and has a property of gelling at 60°C or higher and dissolving at 30°C or lower. In addition, methyl cellulose can be easily formed into a film shape by drying of an aqueous methyl cellulose solution.

Methyl cellulose in the cell culture membrane of the present embodiment has a weight average molecular weight of from 20000 to 190000.

Since the cell culture membrane of the present embodiment contains methyl cellulose having such a weight average molecular weight as a main component, the cell culture membrane can be easily dissolved in a short time of about 10 minutes at a temperature of 15°C or lower.

Specifically, cells are damaged more greatly as they are left in a low-temperature environment for a longer period of time, and therefore, it is desirable to dissolve methyl cellulose in a short time at a temperature that is not low as much as possible.

Therefore, the cell culture membrane of the present embodiment is preferably dissolved at 0°C to 15°C, more preferably at 5°C to 15°C, and still more preferably at 10°C to 15°C.

Examples of the cells to be cultured using the cell culture membrane of the present embodiment include induced pluripotent stem cells (iPS cells and the like) and embryonic stem cells (ES cells).

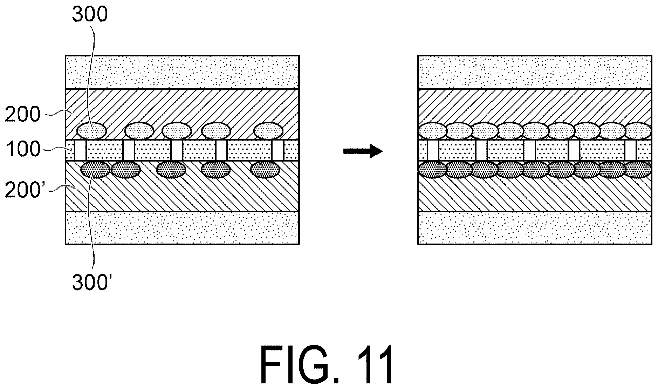

Here, as shown in FIG. 11, a semipermeable membrane made of polyester or the like is generally used as a known cell culture membrane 100 having a cell culture region at both surfaces thereof, and intercellular interaction and exchange of a liquid component are performed between two culture spaces 200 and 200' through the semipermeable membrane.

That is, in the past, the culture space 200 is filled with a culture solution and cells 300 are cultured on one surface of the semipermeable membrane, and the culture space 200' is filled with a culture solution and cells 300' are cultured on the other surface of the semipermeable membrane, so that two cell layers are formed.

However, the size of the cells cultured in the present embodiment is generally 8 to 10 $\mu$m while the pore diameter of the semipermeable membrane is about 3 $\mu$m. Therefore, in the past, there have been issues that the cells are hindered from coming into contact with each other between the cell layers and the interaction between the cell layers is reduced, and that the liquid component exchange efficiency is reduced due to blocking of the pores of the semipermeable membrane along with the proliferation of the cells.

Meanwhile, when a cell culture membrane having a pore diameter of 10 $\mu$m or more is used, cells pass through the cell culture membrane when being seeded, and thus two cell layers cannot be appropriately formed on the cell culture membrane.

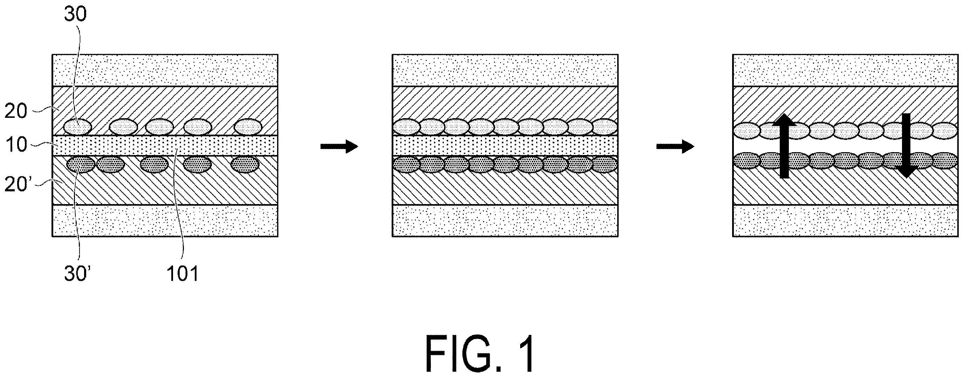

In contrast, as shown in FIG. 1, as for a cell culture membrane 10 of the present embodiment, a culture space 20 is filled with a culture solution and cells 30 are cultured on one surface of the cell culture membrane 10, and a culture space 20' is filled with a culture solution and cells 30' are cultured on the other surface of the cell culture membrane 10, so that two cell layers can be formed.

Then, after the two cell layers are formed, the cell culture membrane 10 is dissolved. As a result, the cells can come into contact with each other between the cell layers, so that the issue of reduction of the interaction between the cell layers can be solved, and the issue of reduction of the liquid component exchange efficiency between the two cell layers can also be solved. The same effect can be achieved also when the cell culture membrane according to a second embodiment described in the following is used.

Second Embodiment

Next, a cell culture membrane according to a second embodiment of the present invention is described.

The cell culture membrane of the present embodiment is a temperature-responsive cell culture base material, contains methyl cellulose having a relatively large weight average molecular weight as a main component, and can be dissolved by being kept at a certain temperature or lower for a predetermined time.

Specifically, the cell culture membrane of the present embodiment has a cell culture region at both surfaces thereof, contains methyl cellulose having a weight average molecular weight (Mw) of from 190000 to 400000 as a main component, and further contains a dissolution temperature regulator. In addition, it is also possible to use methyl cellulose having a weight average molecular weight of from 190000 to 500000 as a main component, it is more preferable to use methyl cellulose having a weight average molecular weight of from 190000 to 390000 as a main component, and it is still more preferable to use methyl cellulose having a weight average molecular weight of from 190000 to 330000 as a main component.

As the dissolution temperature regulator, at least one of sodium styrenesulfonate, sodium iodide, or sodium toluenesulfonate is preferably used.

In the present embodiment, since methyl cellulose having a weight average molecular weight of from 190000 to 400000 is used and the cell culture membrane further contains a dissolution temperature regulator, the cell culture membrane can be easily dissolved in a short time of about 10 minutes at a temperature of 20°C or lower.

The cell culture membrane of the present embodiment is preferably dissolved at 0°C to 20°C, more preferably at 5°C to 20°C, still more preferably at 10°C to 20°C, and particularly preferably at 15°C to 20°C. This is for the purpose of inhibiting damage to cells due to a low-temperature environment as much as possible.

As described above, methyl cellulose has a property of dissolving at 30°C or lower, and the larger the weight average molecular weight is, the longer the period of time during which methyl cellulose has to be kept at a low temperature for dissolution. However, the longer the period of time in the low-temperature environment is, the greater the damage to the cells is. Therefore, it is desirable that the dissolution of methyl cellulose after the temperature drop be completed as quickly as possible, and methyl cellulose is preferably dissolved in 10 minutes or less because the damage to the cells can be inhibited.

Therefore, as described in the section of examples below, the present inventors used methyl cellulose samples having various weight average molecular weights to examine the weight average molecular weight and the dissolution conditions with which methyl cellulose can be dissolved in 10 minutes.

Then, it was found that when the weight average molecular weight of methyl cellulose is in the range of 20000 to 190000, the cell culture membrane can be dissolved in 10 minutes or less by lowering the temperature to 15°C.

Meanwhile, when the weight average molecular weight of methyl cellulose was in the range of 190000 to 400000, the cell culture membrane was not dissolved even when the temperature was lowered to 15°C and held for 10 minutes.

However, it was found that the cell culture membrane can be dissolved in 10 minutes or less by adding a dissolution temperature regulator to the cell culture membrane and lowering the temperature to 20°C.

As described above, with the cell culture membrane of the present embodiment, even when the cell culture membrane contains methyl cellulose having a relatively large weight average molecular weight as a main component, it is possible to dissolve methyl cellulose in the cell culture membrane in 10 minutes or less by adding the dissolution temperature regulator to the cell culture membrane.

In addition, since the cell culture membrane of the present embodiment can be dissolved at a temperature of 20°C, which is higher than the dissolution temperature in the first embodiment, it is possible to further reduce the possibility that the cells in the formed cell layers are damaged.

Third Embodiment

Next, a cell culture membrane according to a third embodiment of the present invention is described.

In the cell culture membrane of the present embodiment, a poorly soluble material is added to the cell culture membrane according to the first embodiment or the cell culture membrane according to the second embodiment. The cell culture membrane of the present embodiment is the same as those of the embodiments in other points.

As the poorly soluble material, a material having cell adhesiveness or a material having no cell adhesiveness can be used.

As the poorly soluble material having cell adhesiveness, for example, polyethylene terephthalate (PET), a glass fiber, a cellulose ester, polyvinylidene fluoride (PVDF), polycarbonate, or the like can be suitably used.

As the poorly soluble material having no cell adhesiveness, polystyrene, a cycloolefin polymer (COP), a cycloolefin copolymer (COC), or the like can be suitably used.

When a material having no cell adhesiveness is used as the poorly soluble material, it is preferable to provide a scaffold material for cells in order to suitably attach the cells to the surface of the cell culture membrane.

That is, it is preferable to add a scaffold material for cells to the surface of the cell culture membrane, and then attach the cells to the cell culture membrane and culture the cells.

As the scaffold material for cells, for example, collagen, Matrigel, fibronectin, laminin, chitosan, silk and the like can be suitably used, and two or more of these materials can also be used in combination.

Examples of the method of adding the scaffold material for cells to the surface of the cell culture membrane include a method of coating the surface with the scaffold material for cells, a method of mixing the scaffold material for cells with the poorly soluble material, a method of chemically bonding the scaffold material for cells to the poorly soluble material using a reagent such as a coupling agent, and a method of attaching collagen or chitosan processed into a fiber in advance to the surface of the cell culture membrane.

In addition, when a material having no cell adhesiveness is used as the poorly soluble material, it is also preferable to subject at least one surface of the cell culture membrane to surface treatment. The surface treatment is preferably corona treatment, excimer treatment, plasma treatment or the like.

Such surface treatment can improve the hydrophilicity of the surface of the cell culture membrane and can improve the adhesiveness of the cells to the surface of the cell culture membrane.

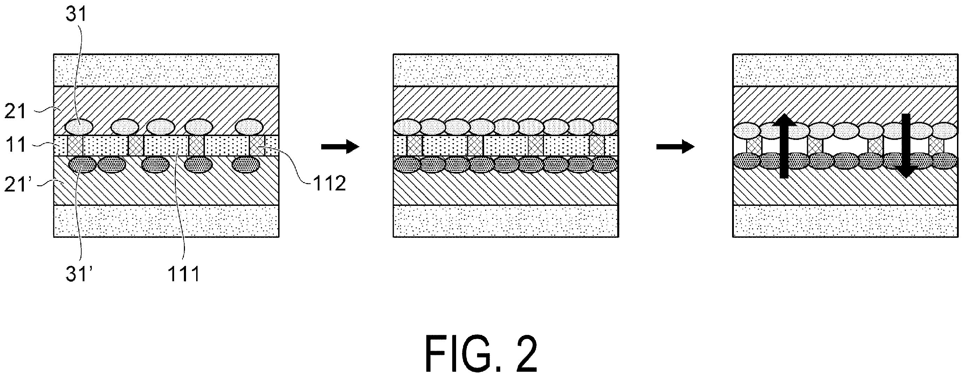

As shown in FIG. 2, as for a cell culture membrane 11 of the present embodiment, a culture space 21 is filled with a culture solution and cells 31 are cultured on one surface of the cell culture membrane 11, and a culture space 21' is filled with a culture solution and cells 31' are cultured on the other surface of the cell culture membrane 11, so that two cell layers can be formed.

Then, methyl cellulose 111 is dissolved after the cell layers are formed, so that only a poorly soluble material 112 in the cell culture membrane 11 is left as a support for the cell layers. As a result, pores penetrating the cell culture membrane 11 are formed.

Specifically, after methyl cellulose 111 is dissolved, the poorly soluble material 112 serves as a support for the cell layers.

In a porous membrane that is formed after the dissolution of methyl cellulose 111 and is made of the poorly soluble material, the pores preferably have a diameter of 10 μm or more. When the pore diameter is set within such a range, the intercellular interaction and the exchange of a liquid component between the two cell layers can be performed with high efficiency.

As described above, with the cell culture membrane 11 of the present embodiment, it is possible to suitably perform the intercellular interaction and the permeation of a liquid component between the plurality of cell layers by dissolving methyl cellulose 111 after the cell layers are formed.

In addition, the poorly soluble material 112 in the cell culture membrane 11 can be left between the plurality of cell layers, and the poorly soluble material 112 can serve as a support to make the cell layers difficult to break.

The cell culture membrane 11 of the present embodiment can be produced by, for example, the following two methods.

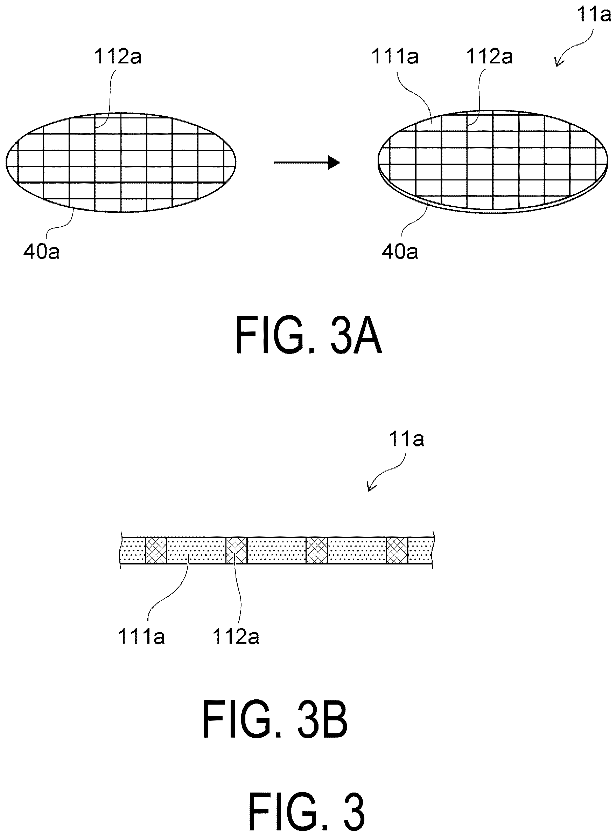

First, in the first method, as shown in FIG. 3A, a support made of a poorly soluble material 112a is produced on a base material 40a. Subsequently, methyl cellulose 111a is applied to the poorly soluble material 112a, whereby a cell culture membrane 11a having the poorly soluble material 112a can be formed on the base material 40a. A cross section of the resulting cell culture membrane 11a is shown in FIG. 3B.

When a cell layer is formed on both surfaces of the cell culture membrane 11a and methyl cellulose 111a is dissolved, the two cell layers are supported by the poorly soluble material 112a, and intercellular interaction and exchange of a liquid component can be performed through pores formed by dissolution of methyl cellulose 111a.

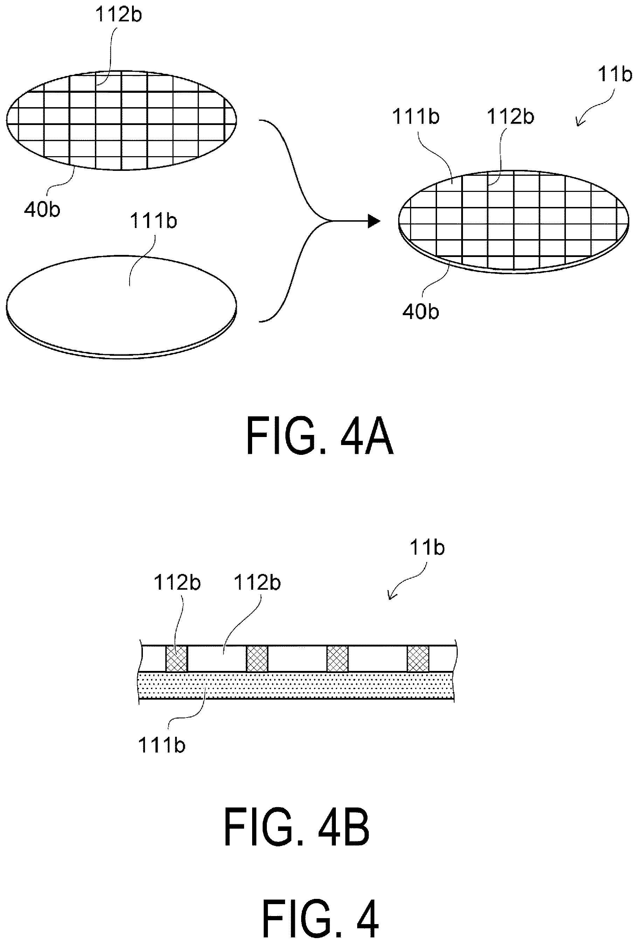

Next, in the second method, as shown in FIG. 4A, a support made of a poorly soluble material 112b is produced on a base material 40b. Separately, a membrane made of methyl cellulose 111b is produced. Then, the support made of the poorly soluble material 112b is removed from the base material 40b and stacked on the membrane made of methyl cellulose 111b, whereby a cell culture membrane 11b having the poorly soluble material 112b can be formed. A cross section of the resulting cell culture membrane 11b is shown in FIG. 4B.

When a cell layer is formed on both surfaces of such a cell culture membrane 11b and methyl cellulose 111b is dissolved, it is possible to achieve a state in which the cell layer formed on the upper side of the cell culture membrane 11b is supported by the poorly soluble material 112b, and the cell layer formed on the lower side of the cell culture membrane 11b is not supported by the poorly soluble material 112b.

With such a cell culture membrane 11b, intercellular interaction and exchange of a liquid component between the two cell layers can be performed more efficiently by dissolution of methyl cellulose 111b.

It is also possible to first form a cell layer only on the upper side of the cell culture membrane 11b, then dissolve methyl cellulose 111b, and then form a cell layer on the lower side of the cell culture membrane 11b.

It is also preferable that the cell culture membrane of each of the above-mentioned embodiments contain adhesive cells. In this case, the adhesive cells may be immobilized on the surface of the cell culture membrane, or the adhesive cells may be embedded in the cell culture membrane.

When the cell culture membrane has such a configuration, it is possible to omit the supply of cells to the culture spaces in the method for producing a biological tissue described below.

Method for Producing Biological Tissue

A method for producing a biological tissue according to an embodiment of the present invention is a method for producing a biological tissue including a plurality of cell layers, and includes:

(A1) supplying cells and a culture solution to two culture spaces of a biological tissue forming device including a cell culture membrane and the two culture spaces, the cell culture membrane having a cell culture region at both surfaces thereof, the cell culture membrane being disposed between a plurality of cell layers after the cells are cultured, and the cell culture membrane further containing methyl cellulose having a weight average molecular weight of from 20000 to 190000 as a main component, and the two culture spaces being divided by the cell culture membrane;

(A2) culturing the cells in the two culture spaces to form cell layers on both surfaces of the cell culture membrane; and

(A3) dissolving methyl cellulose in the cell culture membrane at 15°C or lower.

It is also preferable that the method for producing a biological tissue according to the present embodiment includes:

(B1) supplying cells and a culture solution to two culture spaces of a biological tissue forming device including a cell culture membrane and the two culture spaces, the cell culture membrane having a cell culture region at both surfaces thereof, the cell culture membrane being disposed between a plurality of cell layers after the cells are cultured, the cell culture membrane containing methyl cellulose having a weight average molecular weight of from 190000 to 400000 as a main component, and the cell culture membrane further containing a dissolution temperature regulator, and the two culture spaces being divided by the cell culture membrane;

(B2) culturing the cells in the two culture spaces to form cell layers on both surfaces of the cell culture membrane; and

(B3) dissolving methyl cellulose in the cell culture membrane at 20°C or lower.

Furthermore, it is also preferable that the method for producing a biological tissue according to the present embodiment have the following configuration.

That is, it is preferable that, in each of (A3) and (B3), methyl cellulose in the cell culture membrane be dissolved in 10 minutes or less.

In addition, it is preferable to employ a method in which a poorly soluble material is added to the cell culture membrane, and the cell layers are supported by the poorly soluble material after dissolution of methyl cellulose in cell culture membrane.

As the cell culture membrane used in the method for producing a biological tissue of the present embodiment, any of the cell culture membranes in the above-mentioned embodiments can be used. In addition, cells same as those in the above-mentioned embodiments can be used as the cells to be cultured.

In (A3) of the method for producing a biological tissue of the present embodiment, methyl cellulose is preferably dissolved at 0°C to 15°C, more preferably at 5°C to 15°C, and still more preferably at 10°C to 15°C.

This is because cells are damaged more greatly as they are left in a low-temperature environment for a longer period of time, and therefore, it is desirable to dissolve methyl cellulose in a short time at a temperature that is not low as much as possible.

For the same reason, in (B3) of the method for producing a biological tissue of the present embodiment, methyl cellulose is preferably dissolved at 0°C to 20°C, more preferably at 5°C to 20°C, still more preferably at 10°C to 20°C, and particularly preferably at 15°C to 20°C.

In the method for producing a biological tissue of the present embodiment, as the biological tissue forming device, for example, an organ chip, a microfluidic device, or the like can be suitably used.

However, the method for producing a biological tissue of the present embodiment is not limited to the cases in which these devices are used, and includes cases in which any other devices are used as long as the cell culture membrane of each of the above-mentioned embodiments is used in the method.

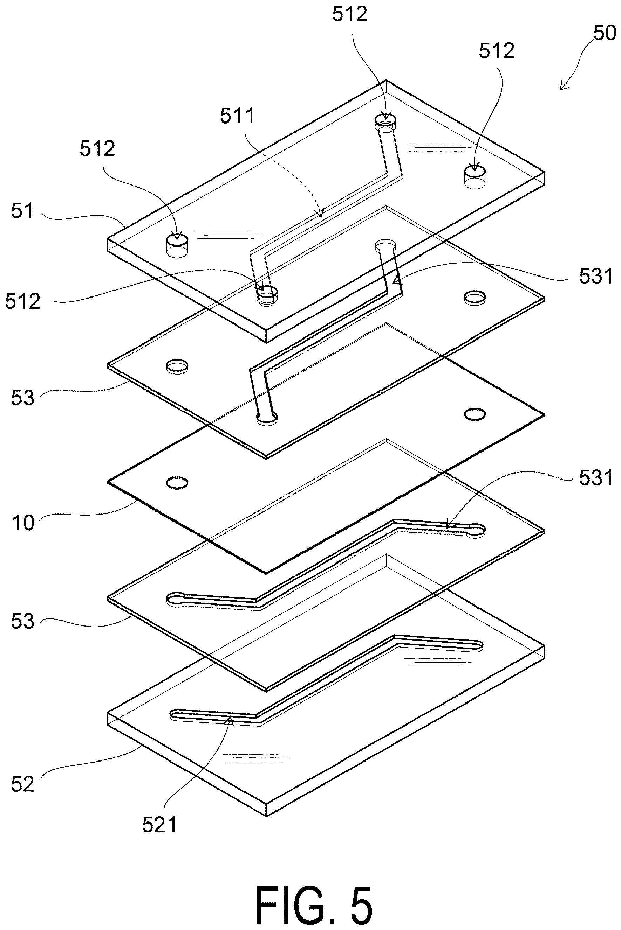

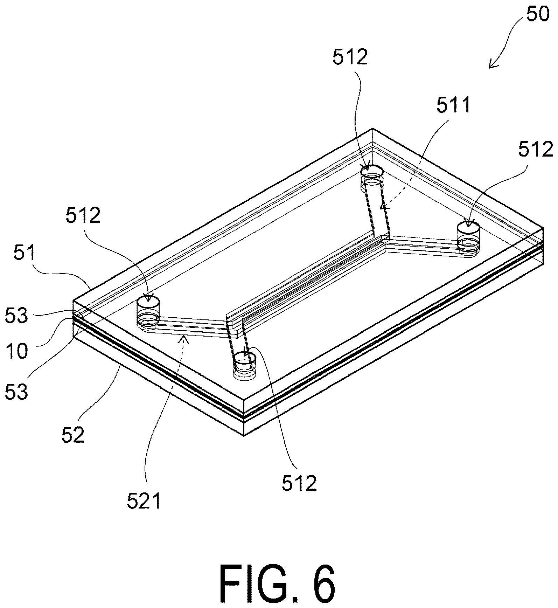

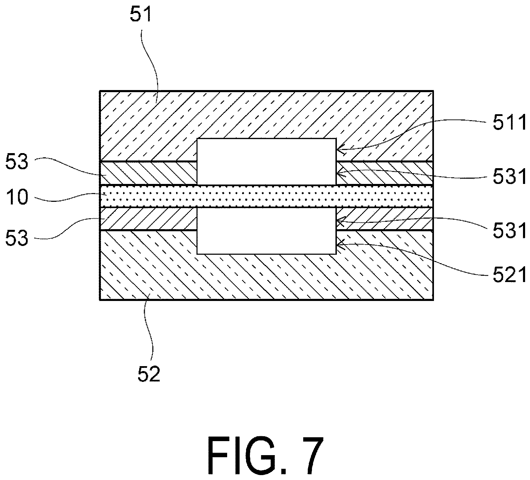

In the method for producing a biological tissue of the present embodiment, when a microfluidic device is used as the biological tissue forming device, for example, the one shown in FIGS. 5 to 7 can be used. FIG. 7 shows a partial cross section taken at the center of a cross section of a microfluidic device 50 of the present embodiment shown in FIG. 6, which is obtained by vertically cutting the microfluidic device 50 at the center of the long axis direction thereof.

Specifically, the microfluidic device 50, in which the cell culture membrane of each of the above-mentioned embodiments can be used, can be formed by stacking an upper substrate 51, the cell culture membrane 10 (or the cell culture membrane 11), and a lower substrate 52.

The upper substrate 51 has a flow path 511, the lower substrate 52 has a flow path 521, and these flow paths are separated from each other by the cell culture membrane 10.

The upper substrate 51 and the cell culture membrane 10 are bonded to each other by an adhesive sheet 53, and the lower substrate 52 and the cell culture membrane 10 are bonded to each other by another adhesive sheet 53. In addition, the adhesive sheets 53 individually have a flow path 531 at positions corresponding to the flow paths 511 and 521.

In the microfluidic device 50 of the present embodiment, each of the upper substrate 51, the cell culture membrane 10, and the adhesive sheet 53 bonding the upper substrate 51 to the cell culture membrane 10 has liquid feed holes for feeding a liquid to the flow paths 511 and 521, and a culture medium, a reagent, or the like can be fed through the liquid feed holes.

Use of the cell culture membrane of each of the above-mentioned embodiments in such a microfluidic device 50 makes it possible to suitably produce various biological tissues. For example, it is possible to produce various biological tissues such as biological tissues in a proximal tubule model including a tissue formed of tubular epithelial cells and a tissue formed of vascular endothelial cells, and biological tissues in a glomerulus model, a small intestine model, a liver model, and a lung model.

As described above, with the cell culture membrane and the method for producing a biological tissue of the present embodiment, the cell culture membrane can be easily decomposed after the cell layers are formed on both sides of the cell culture membrane. For this reason, it is possible to inhibit the hindrance of intercellular interaction and permeation of a liquid component between the cell layers.

In addition, with the cell culture membrane and the method for producing a biological tissue of the present embodiment, it is possible to dissolve the cell culture membrane in a short time of 10 minutes or less for any cell culture membrane containing methyl cellulose having weight average molecular weights in different ranges. Therefore, in the formed cell layers, it is possible to inhibit damage to the cells due to a low-temperature environment.

Examples

Hereinafter, experiments performed to confirm the dissolution conditions of the cell culture membranes according to the embodiments of the present invention are described.

Experiment 1

First, an experiment for confirming conditions applicable to the cell culture membrane of the present embodiment was performed using methyl cellulose having various weight average molecular weights.

Specifically, five types of methyl cellulose samples having different viscosities were provided. The sample names were MC-4 (viscosity η = from 3.2 to 4.8, manufactured by Shin-Etsu Chemical Co., Ltd., MCE-4), MC-400 (viscosity η = from 280 to 560, manufactured by Shin-Etsu Chemical Co., Ltd., MCE-400), MC-1500 (viscosity η = from 1000 to 1800, manufactured by Tokyo Chemical Industry Co., Ltd., M0294), MC-4000 (viscosity η = from 2800 to 5600, manufactured by Shin-Etsu Chemical Co., Ltd., MCE-4000), and MC-10000 (viscosity η = from 7000 to 10000, manufactured by Tokyo Chemical Industry Co., Ltd., M0295).

In addition, the weight average molecular weight Mw of each sample was calculated based on the viscosity η (mPa·s) using the following equation.

$$\text{Weight average molecular weight Mw} = 40000 \times (\text{Log}\eta) + 880 \times (\text{Log}\eta)^4$$

As a result, the weight average molecular weight of MC-4 was from 20263 to 27439, the weight average molecular weight of MC-400 was from 129446 to 160123, the weight average molecular weight of MC-1500 was from 191280 to 229028, the weight average molecular weight of MC-4000 was from 262145 to 323615, and the weight average molecular weight of MC-10000 was from 346163 to 385280.

The weight average molecular weight corresponding to the viscosity η = 4 was 24198, the weight average molecular weight corresponding to the viscosity η = 400 was 144424, the weight average molecular weight corresponding to the viscosity η = 1500 was 216591, the weight average molecular weight corresponding to the viscosity η = 4000 was 292227, and the weight average molecular weight corresponding to the viscosity η = 10000 was 385280.

Next, 20 mL of 2% aqueous solutions of each methyl cellulose were prepared and used as samples without an additive. In addition, sodium styrenesulfonate (NaSS, manufactured by Fujifilm Wako Pure Chemical Industries, Ltd., 192-03292) was added to 20 mL of 2% aqueous solutions of each methyl cellulose so that the resulting samples had a concentration of 0.1 M, which were used as samples with an additive.

Then, each of the solutions was poured into a 1.5 cm square frame and dried to prepare the thickness, whereby a 1.5 cm square cell culture membrane was produced for each sample. The thickness of each sample was targeted to be about 10 to 30 μm.

Each sample was stored in warm water at 37°C for one week to swell, and then taken out of the warm water and allowed to stand at 15°C for 10 minutes, and the dissolution state of each sample was visually observed. Immediately after being taken out of the warm water, each sample maintained the state of the 1.5 cm square membrane.

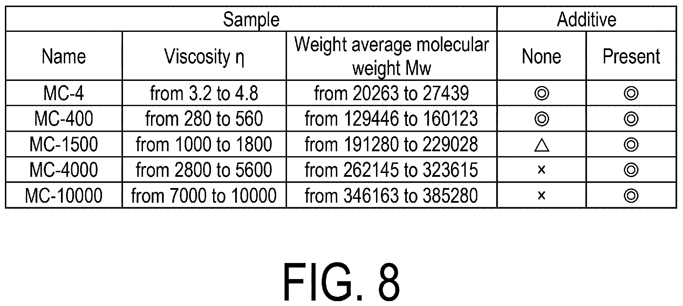

The results of Experiment 1 are shown in FIG. 8. In the table, the case where the sample was completely dissolved is indicated by ◎, the case where there was an undissolved residue in a gel form is indicated by Δ, and the case where there was an undissolved residue in a membrane form is indicated by ×. This also applies to the results of the following experiments.

As shown in FIG. 8, in the case without an additive, the samples MC-4 and MC-400 were dissolved, but the samples MC-1500, MC-4000, and MC-10000 were not sufficiently dissolved. On the other hand, with an additive, all the samples were dissolved.

That is, it was possible to dissolve methyl cellulose having a weight average molecular weight of from about 20000 to 190000 within 10 minutes at 15°C in the case without an additive.

In addition, it was possible to dissolve methyl cellulose having a weight average molecular weight of from about 190000 to 400000 within 10 minutes at 15°C in the case with an additive.

Experiment 2

Next, an experiment for confirming temperature conditions capable of dissolving the cell culture membrane of the present embodiment was performed using methyl cellulose having various weight average molecular weights.

In this experiment, four types of methyl cellulose samples, that is, MC-4, MC-400, MC-1500, and MC-4000 were provided, and three samples without an additive and three samples with an additive were prepared in the same manner as in Example 1.

Then, each sample was stored in warm water at 37°C for one week to swell, and then taken out of the warm water and allowed to stand at the temperatures of 15°C, 20°C, and 25°C each for 10 minutes, and the dissolution state of each sample was visually observed.

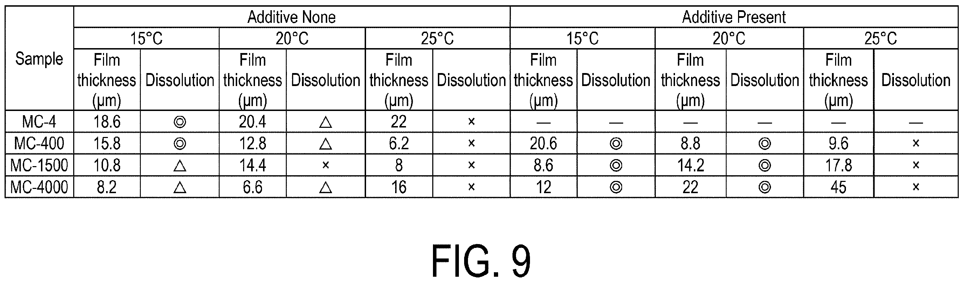

The results of Experiment 2 are shown in FIG. 9. As shown in the table, in the case without an additive, when the samples were allowed to stand at 15°C for 10 minutes, the samples MC-4 and MC-400 were dissolved, but the samples MC-1500 and MC-4000 were not sufficiently dissolved. Further, when the samples were allowed to stand at 20°C or 25°C for 10 minutes, all the samples were not sufficiently dissolved.

On the other hand, with an additive, all the samples MC-400, MC-1500, and MC-4000 were dissolved when the samples were allowed to stand at 15°C or 20°C for 10 minutes. Further, when the samples were allowed to stand at 25°C for 10 minutes, all the samples were not sufficiently dissolved.

That is, it was possible to dissolve methyl cellulose having a weight average molecular weight of from about 20000 to 190000 in 10 minutes at 15°C in the case without an additive.

In addition, it was possible to dissolve methyl cellulose having a weight average molecular weight of from about 190000 to 330000 in 10 minutes at 15°C and 20°C in the case with an additive.

Experiment 3

Further, an experiment was performed under various concentrations of the additive contained in the cell culture membrane of the present embodiment to confirm whether the cell culture membrane can be dissolved.

In this experiment, MC-4000 was provided as methyl cellulose, and sodium styrenesulfonate (NaSS, manufactured by Fujifilm Wako Pure Chemical Industries, Ltd., 192-03292) was added to 20 mL of 2% aqueous solutions of methyl cellulose so that the resulting samples had a concentration of 0.05 M, 0.1 M, 0.3 M, or 0.5 M to prepare samples in the same manner as in Example 1.

Then, each sample was stored in warm water at 37°C for one week to swell, and then taken out of the warm water and allowed to stand at the temperatures of 15°C and 20°C each for 10 minutes, and the dissolution state of each sample was visually observed.

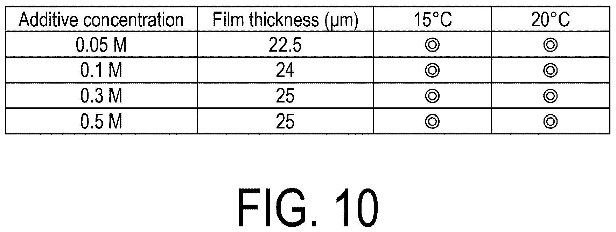

The results of Experiment 3 are shown in FIG. 10. As shown in the table, all the samples were dissolved.

Here, in the sample to which the additive was added at a concentration of 0.5 M, the cell culture membrane was fragile and was cumbersome to handle. Therefore, it was found that the concentration of the additive contained in the cell culture membrane can be set to 0.05 M to 0.5 M, and is preferably 0.05 M to 0.3 M, and more preferably 0.05 M to 0.2 M.

The present invention is not limited only to the embodiments and examples described above, and it goes without saying that various modifications can be made within the scope of the present invention.

For example, it is possible to make appropriate modifications, such as employing a method other than the above-mentioned method as the method for producing a cell culture membrane to which a poorly soluble material is added, or using the cell culture membrane in a biological tissue forming device other than a microfluidic device.

Industrial Applicability

The present invention can be suitably used, for example, in a case where a biological tissue including a plurality of cell layers is produced.

The contents of the literature described in this description and the description of the Japanese application that is the basis of Paris priority of the present application are all incorporated herein.

Reference Signs List

10, 11, 11a, 11b Cell culture membrane
101, 111, 111a, 111b Methyl cellulose
112, 112a, 112b Poorly soluble material
20, 20', 21, 21' Culture space
30, 30', 31, 31' Cell
40a, 40b Base material
50 Microfluidic device
51 Upper substrate
511 Flow path
512 Liquid feed hole
52 Lower substrate
521 Flow path
53 Adhesive sheet
531 Flow path

**Claims**

1.  A cell culture membrane having a cell culture region at both surfaces thereof, the cell culture membrane comprising methyl cellulose having a weight average molecular weight of from 20000 to 190000 as a main component.

2.  A cell culture membrane having a cell culture region at both surfaces thereof, the cell culture membrane comprising methyl cellulose having a weight average molecular weight of from 190000 to 400000 as a main component, and further comprising a dissolution temperature regulator.

3.  The cell culture membrane according to claim 2, wherein the dissolution temperature regulator is at least one of sodium styrenesulfonate, sodium iodide, or sodium toluenesulfonate.

4.  The cell culture membrane according to any one of claims 1 to 3, wherein a poorly soluble material is added to the cell culture membrane.

5.  The cell culture membrane according to claim 4, wherein the poorly soluble material is at least one of polyethylene terephthalate, a glass fiber, a cellulose ester, polyvinylidene fluoride, or polycarbonate.

6.  The cell culture membrane according to claim 4, wherein the poorly soluble material is at least one of polystyrene, a cycloolefin polymer, or a cycloolefin copolymer.

7.  The cell culture membrane according to claim 6, wherein the cell culture membrane has a scaffold material for cells on at least one surface thereof.

8.  The cell culture membrane according to claim 7, wherein the scaffold material is at least one of collagen, Matrigel, fibronectin, laminin, chitosan, or silk.

9.  The cell culture membrane according to claim 6, wherein at least one surface of the cell culture membrane is subjected to surface treatment.

10. The cell culture membrane according to any one of claims 1 to 9, further comprising adhesive cells.

11. A method for producing a biological tissue including a plurality of cell layers, the method comprising:

    supplying cells and a culture solution to two culture spaces of a biological tissue forming device including a cell culture membrane and the two culture spaces, the cell culture membrane having a cell culture region at both surfaces thereof, the cell culture membrane being disposed between a plurality of cell layers after the cells are cultured, and the cell culture membrane further containing methyl cellulose having a weight average molecular weight of from 20000 to 190000 as a main component, and the two culture spaces being divided by the cell culture membrane;
    culturing the cells in the two culture spaces to form cell layers on both surfaces of the cell culture membrane; and
    dissolving methyl cellulose in the cell culture membrane at 15°C or lower.

12. A method for producing a biological tissue including a plurality of cell layers, the method comprising:

    supplying cells and a culture solution to two culture spaces of a biological tissue forming device including a cell culture membrane and the two culture spaces, the cell culture membrane having a cell culture region at both surfaces thereof, the cell culture membrane being disposed between a plurality of cell layers after the cells are cultured, the cell culture membrane containing methyl cellulose having a weight average molecular weight of from 190000 to 400000 as a main component, and the cell culture membrane further containing a dissolution temperature regulator, and the two culture spaces being divided by the cell culture membrane;
    culturing the cells in the two culture spaces to form cell layers on both surfaces of the cell culture membrane; and
    dissolving methyl cellulose in the cell culture membrane at 20°C or lower.

13. The method for producing a biological tissue according to claim 11 or 12, wherein methyl cellulose in the cell culture membrane is dissolved in 10 minutes or less.

14. The method for producing a biological tissue according to any one of claims 11 to 13, wherein a poorly soluble material is added to the cell culture membrane, and the cell layers are supported by the poorly soluble material after dissolution of methyl cellulose.

# FIG. 1

FIG. 2

EP 4 455 262 A1

112a

111a    112a    11a

40a    40a

# FIG. 3A

11a

111a    112a

# FIG. 3B

# FIG. 3

112b

111b    112b    11b

40b

111b

40b

# FIG. 4A

112b    112b    11b

111b

# FIG. 4B

# FIG. 4

# FIG. 5

FIG. 6

FIG. 7

| Sample | | | Additive | |
| --- | --- | --- | --- | --- |
| Name | Viscosity η | Weight average molecular weight Mw | None | Present |
| MC-4 | from 3.2 to 4.8 | from 20263 to 27439 | ◎ | ◎ |
| MC-400 | from 280 to 560 | from 129446 to 160123 | ◎ | ◎ |
| MC-1500 | from 1000 to 1800 | from 191280 to 229028 | △ | ◎ |
| MC-4000 | from 2800 to 5600 | from 262145 to 323615 | × | ◎ |
| MC-10000 | from 7000 to 10000 | from 346163 to 385280 | × | ◎ |

# FIG. 8

| Sample | Additive None | | | | | | Additive Present | | | | | |
|---|---|---|---|---|---|---|---|---|---|---|---|---|
| | 15°C | | 20°C | | 25°C | | 15°C | | 20°C | | 25°C | |
| | Film thickness (μm) | Dissolution | Film thickness (μm) | Dissolution | Film thickness (μm) | Dissolution | Film thickness (μm) | Dissolution | Film thickness (μm) | Dissolution | Film thickness (μm) | Dissolution |
| MC-4 | 18.6 | ◎ | 20.4 | △ | 22 | × | — | — | — | — | — | — |
| MC-400 | 15.8 | ◎ | 12.8 | △ | 6.2 | × | 20.6 | ◎ | 8.8 | ◎ | 9.6 | × |
| MC-1500 | 10.8 | △ | 14.4 | × | 8 | × | 8.6 | ◎ | 14.2 | ◎ | 17.8 | × |
| MC-4000 | 8.2 | △ | 6.6 | △ | 16 | × | 12 | ◎ | 22 | ◎ | 45 | × |

# FIG. 9

| Additive concentration | Film thickness (μm) | 15°C | 20°C |
|---|---|---|---|
| 0.05 M | 22.5 | ◎ | ◎ |
| 0.1 M | 24 | ◎ | ◎ |
| 0.3 M | 25 | ◎ | ◎ |
| 0.5 M | 25 | ◎ | ◎ |

# FIG. 10

300

200

100

200'

300'

FIG. 11

## INTERNATIONAL SEARCH REPORT

| International application No. |
|---|
| **PCT/JP2022/042045** |

### A. CLASSIFICATION OF SUBJECT MATTER

*C12M 3/00*(2006.01)i
FI:   C12M3/00 A

According to International Patent Classification (IPC) or to both national classification and IPC

### B. FIELDS SEARCHED

Minimum documentation searched (classification system followed by classification symbols)

C12M3/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN); PubMed

### C. DOCUMENTS CONSIDERED TO BE RELEVANT

| Category* | Citation of document, with indication, where appropriate, of the relevant passages | Relevant to claim No. |
|---|---|---|
| X | JP 2012-115262 A (NATIONAL INSTITUTE OF AGROBIOLOGICAL SCIENCES) 21 June 2012 (2012-06-21) claims 7, 16, paragraphs [0031], [0032] | 1 |
| Y | claims 7, 16, paragraphs [0031], [0032] | 4-10 |
| Y | JP 2017-504320 A (PRESIDENT AND FELLOWS OF HARVARD COLLEGE) 09 February 2017 (2017-02-09) claims 1, 29, 30, 53-55, 94, paragraphs [0131], [0318] | 4-10 |
| A | US 2018/0230415 A1 (THE TRUSTEES OF THE UNIVERSITY OF PENNSYLVANIA) 16 August 2018 (2018-08-16) abstract, paragraphs [0006], [0083], [0086], [0088], [0089], fig. 1-3 | 1-14 |
| A | JP 2008-263863 A (DAINIPPON PRINTING CO., LTD.) 06 November 2008 (2008-11-06) paragraphs [0049], [0050] | 1-14 |
| A | JP 2020-028305 A (NATIONAL AGRICULTURE AND FOOD RESEARCH ORGANIZATION) 27 February 2020 (2020-02-27) paragraphs [0022], [0026], fig. 2 | 1-14 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 January 2023** | **31 January 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

# EP 4 455 262 A1

**INTERNATIONAL SEARCH REPORT**

| International application No. |
| --- |
| **PCT/JP2022/042045** |

**C. DOCUMENTS CONSIDERED TO BE RELEVANT**

| Category* | Citation of document, with indication, where appropriate, of the relevant passages | Relevant to claim No. |
| --- | --- | --- |
| P, X | WO 2021/261495 A1 (TOYO SEIKAN GROUP HOLDINGS LTD.) 30 December 2021 (2021-12-30)<br>paragraphs [0028]-[0034], [0064]-[0066], fig. 4 | 1-14 |

Form PCT/ISA/210 (second sheet) (January 2015)

**INTERNATIONAL SEARCH REPORT**
Information on patent family members

International application No.

**PCT/JP2022/042045**

| Patent document cited in search report | | | Publication date (day/month/year) | Patent family member(s) | | | Publication date (day/month/year) |
|---|---|---|---|---|---|---|---|
| JP | 2012-115262 | A | 21 June 2012 | US claims 7, 16, paragraphs [0093], [0094] | 2013/0280807 | A1 | |
| | | | | WO | 2012/063925 | A1 | |
| | | | | EP | 2639293 | A1 | |
| | | | | CN | 103261394 | A | |
| JP | 2017-504320 | A | 09 February 2017 | US claims 1, 29, 30, 53-55, 94, paragraphs [0167], [0354] | 2016/0313306 | A1 | |
| | | | | WO | 2015/138034 | A2 | |
| | | | | EP | 3083940 | A2 | |
| | | | | CN | 106459898 | A | |
| US | 2018/0230415 | A1 | 16 August 2018 | WO abstract, p. 2, lines 6-12, p. 17, lines 31, 32, p. 18, line 26 to p. 19, line 2, p. 19, line 22 to p. 20, line 5, fig. 1-3 | 2017/019796 | A1 | |
| JP | 2008-263863 | A | 06 November 2008 | US paragraphs [0099], [0100] | 2008/0227203 | A1 | |
| | | | | EP | 1970439 | A2 | |
| | | | | EP | 2135940 | A1 | |
| JP | 2020-028305 | A | 27 February 2020 | US fig. 2 | 2018/0120298 | A1 | |
| | | | | WO | 2016/158417 | A1 | |
| | | | | EP | 3275994 | A1 | |
| WO | 2021/261495 | A1 | 30 December 2021 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017504320 T